(19) Europäisches Patentamt
European Patent Office
Office européen des brevets

(11) EP 1 370 541 B1

(12) **EUROPEAN PATENT SPECIFICATION**

(45) Date of publication and mention of the grant of the patent:
**02.11.2006 Bulletin 2006/44**

(21) Application number: **01919747.4**

(22) Date of filing: **20.02.2001**

(51) Int Cl.:
***C07D 263/06*** *(2006.01)*

(86) International application number:
**PCT/IN2001/000020**

(87) International publication number:
**WO 2002/066448 (29.08.2002 Gazette 2002/35)**

# (54) METHOD OF PREPARATION OF PACLITAXEL (TAXOL) USING 3-(ALK-2-YNYLOXY) CARBONYL-5-OXAZOLIDINE CARBOXYLIC ACID

VERFAHREN ZUR HERSTELLUNG VON PACLITAXEL (TAXOL) UNTER VERWENDUNG VON 3-(ALK-2-INYLOXY)CARBONYL-5-OXAZOLIDINCARBONSÄURE

PROCEDE DE PREPARATION DE PACLITAXEL (TAXOL) UTILISANT UN ACIDE 3-(ALK-2-YNYLOXY) CARBONYL-5-OXAZOLIDINE CARBOXYLIQUE

(84) Designated Contracting States:
**AT BE CH CY DE DK ES FI FR GB GR IE IT LI LU MC NL PT SE TR**

(43) Date of publication of application:
**17.12.2003 Bulletin 2003/51**

(73) Proprietor: **Dabur India Limited**
**Kalyani,**
**Nadia 741235 (IN)**

(72) Inventors:
- **SHARMA, Arun, Prakash,**
**Dabur India Limited**
**Kalyani,**
**Nadia 741235 (IN)**
- **SARKAR, Subrata,**
**Dabur India Limited**
**Kalyani,**
**Nadia 741235 (IN)**

(74) Representative: **Petersen, Frank et al**
**Lemcke, Brommer & Partner**
**Patentanwälte**
**Bismarckstrasse 16**
**76133 Karlsruhe (DE)**

(56) References cited:
**WO-A1-93/16060**
**WO-A1-94/07878**
**US-A- 5 811 550**

EP 1 370 541 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

FIELD OF THE INVENTION

The present invention relates to a novel oxazolidine carboxylic acid and a process for its preparation. The invention further relates to a process for the preparaton of paclitaxel (taxol) using such oxazolidine carboxylic acid. Paclitaxel (Taxol) (1) is a terpene of taxane family and has the formula shown below and has an application for treatment of various types of cancer. Non-natural analog of paclitaxel (taxol), docetaxel (taxotere) (2) is also an approved anticancer drug. Thus, there is great interest in molecules having similar structures for development of new anticancer drugs and SAR studies.

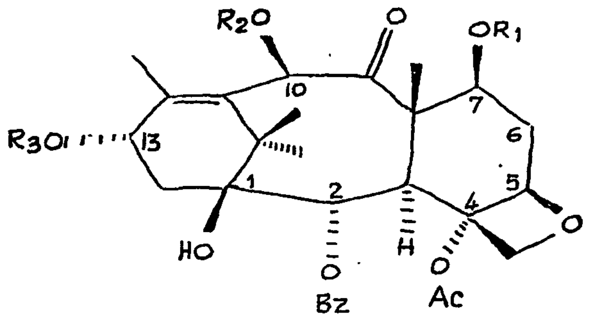

1. Taxol $R_1$=H, $R_2$=Ac, $R_3$= 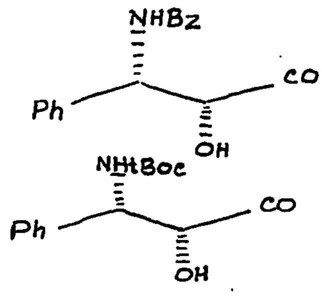

2. Taxotere $R_1$=$R_2$=H, $R_3$=

3. Baccatin $R_1$=$R_3$=H, $R_2$=Ac

4. 10-Deacetylbacctin (DAB) $R_1$=$R_2$=$R_3$=H

BACKGROUND OF THE INVENTION

The structure of paclitaxel (taxol) has two distinct units. One is baccatin (3) and other is N-benzoylphenylisoserine, the side chain, connected to baccatin at C-13 through ester linkage. It has been shown that the β-amido-α-hydroxy ester side chain at C-13 is very essential for anticancer activity of taxol and any other taxol analogs. The supply of taxol from natural sources is very limited and so there is great interest in its synthesis. 10-deacetyl baccatin (4) is more readily available than taxol from the leaves of yew tree and comprises a starting material for semisynthesis of taxol and taxol analogs. It is known in the literature that it is very difficult to esterify 13 hydroxy of bacctin with β-amido-α-hydroxy carboxylic acid. The difficulty has been ascribed to spatial disposition of 13 hydroxy in the baccatin nucleus. Therefore, considerable efforts have been made to find precursors of β-amido-α-hydroxy carboxylic acid which can be coupled with bacctin, in high yield and the resultant couple product can be processed to afford 13-0- (β-amido-α-hydroxycarbonyl) baccatin in high yield and purity.

The following types of cyclic derivative of phenylisoserine are known to couple with 13-hydroxy of baccatin.

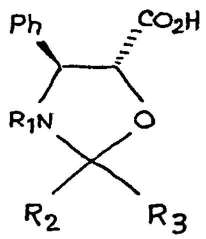

5

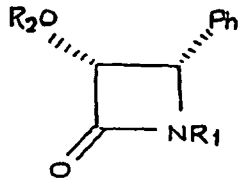

6

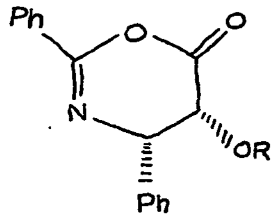

7

R, $R_1$, $R_2$, and $R_3$ = Protecting groups

In the literature, various protecting groups, $R_1$, are described in oxazolidinecarboxylic acid (5). For example, US 5, 811, 550, WO 93/16060 and WO 94/07878 describe such compounds. However, alk-2-ynyloxycarbonyl group have not been described so far. The utility of this group over other commonly used protecting groups lies in the fact that this group can be cleaved under neutral condition. All other known protecting groups ($R_1$) are cleaved under either acidic conditions or by hydrogenolysis. The subsequent cleavage of protecting groups $R_2$ and $R_3$ are normally very fast, once $R_1$ is cleaved. Since the baccatin part of taxol is very prone to degradation even under mild acidic or basic conditions, removal of alk-2-ynyloxycarbonyl group under neutral condition facilitates the conversion of taxol intermediate such as 10 to taxol in high yield and high purity.

OBJECTS OF THE INVENTION

An object of this invention is to propose a novel oxazolidine carboxylic acid and a process for the preparation thereof.

Another object of this invention is to propose oxazolidine carboxylic acid which can advantageously be used in the preparation of anticancer drugs.

Still another object of this invention is to propose a process for the preparation of taxol and its synthetic analogs using the proposed oxazolidine carboxylic acid.

DESCRIPTION OF THE INVENTION

The present invention is directed to the oxazolidine carboxylic acid having a formula wherein $R_1$, $R_2$ and $R_3$ are hydrogen, $R_4$ is phenyl and $R_5$ and $R_6$ are methyl.

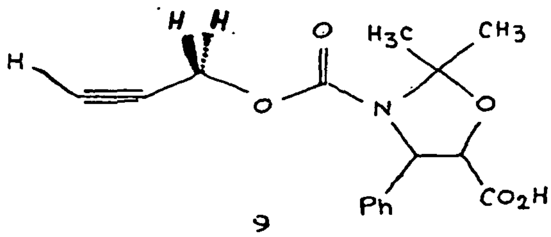

9

The present invention also describes a process of preparation of oxazolidine carboxylic acid.

The process is illustrated in reaction 1:

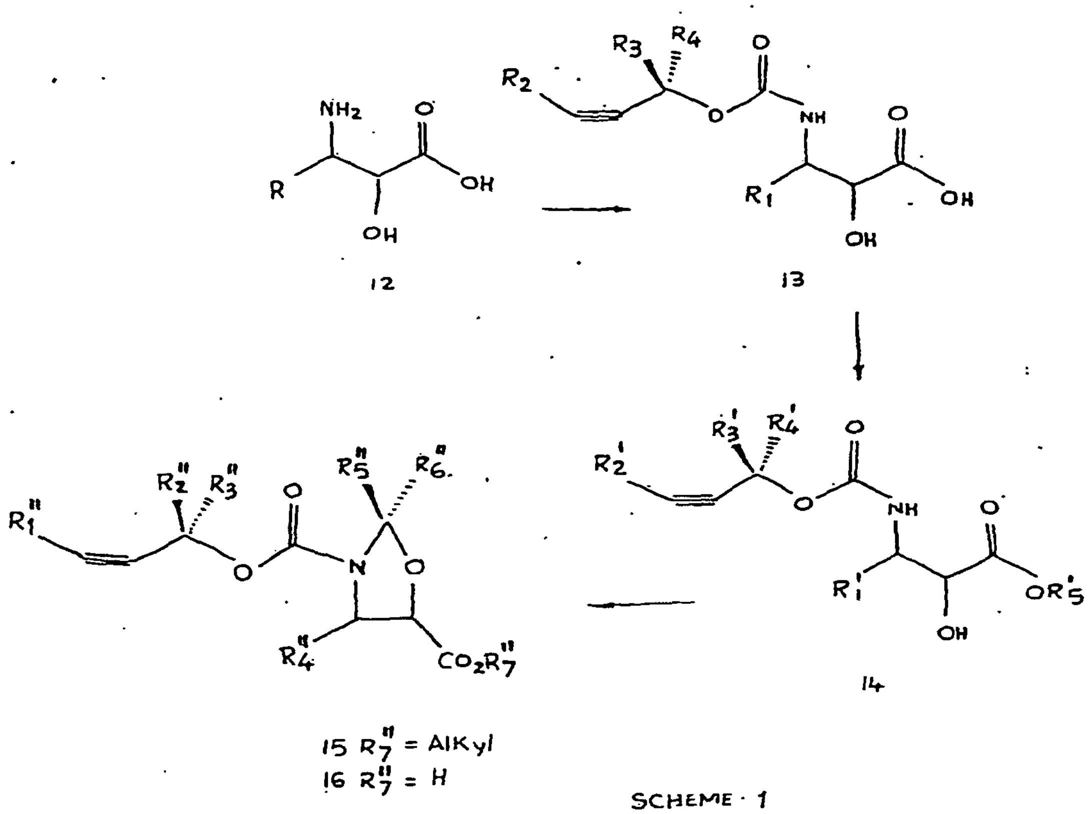

12

13

14

15 $R''_7$ = Alkyl

16 $R''_7$ = H

SCHEME 1

In this process, isoserine 12 is the starting material. In the preferred starting material, R is aryl and the most preferred R is phenyl. The arylisoserine has two asymmetric carbons, all optically active forms such as enantiomers, diastereomers, racemic mixtures and other mixtures are contemplated here. The preferred optically active form of pheneylisoserine is 2R, 3S. This phenylisoserine is commercially available.

Thus, phenylisoserine is condensed with alk-2-ynyl haloformate in presence of some base such as alkali hydroxide or carbonate or bicarbonate or any other acid neutralising chemical. Herein, alk-2-ynyl groups, either alone or with the various substitutents defined above are preferably lower alk-2-ynyl containing from three to six carbon atoms in the principal chain and up to 10 carbon atoms and include but-2-ynyl, pent-2-ynyl, prop-2 ynyl, hex-2-ynyl and the like.

The most preferred haloformate is prop-2-ynyl chloroformate which is available commercially. This can be prepared from diphosgene/triphosgene and propargyl alcohol following the procedure given in Helv.Chim.Acta. Vol 77, 561, 1994.

The most preferred base here is sodium bicarbonate. The condensation is carried out by addition of chloroformate into a solution of isoserine in aqueous bicarbonate over a period of 5-30 min, most preferably over 10-15 min. The acidification of the reaction mixture affords the N-(alk-2-ynyloxy)carbonylisoserine 13. The most preferred one is where $R_1$ is phenyl, $R_2$, $R_3$, and $R_4$ are hydrogen.

N-(Alk-2-ynyloxy)carbonylisoserine is then converted into corresponding ester in presence of an alcohol and an activating agent such as dicyclohexylcarbodiimide or carbonyldiimidazole. The most preferred alcohol is methanol and the most preferred activating agents is carbonyldiimidazole. The esterification can be carried out in various solvents such as dichloromethane, chloroform, ethyl acetate, tetrahydrofuran, acetonitrile or dimethylformamide. The most preferred solvent is acetonitrile. The esterification is carried out by first mixing acid with activating agent for 0.5-6 hr, most preferably for 1 hr, at 0-25°C, most preferably at 10°C. Then the alcohol is introduced in the mixture over a period of 10-60 min, most preferably over 30 min and the mixing is done for 1-6 hr, most preferably for 2 hr.

Methyl ester is alternatively prepared by condensing isoserine with diazomethane. The ethereal solution of diazomethane is prepared from following the standard procedure described in Vogel's Text book of Practical Organic Chemistry, 1989, page 432. The methylation is effected by mixing the solution of N-(alk-2-ynyloxy)carbonylisoserine in tetrahydrofuran with excess ethereal solution of diazomethane. The resultant mixture is stored for 1-6 hr, most preferably for 2 hr at 5-25°C, most preferably at 10°C, for complete reaction.

In the most preferred ester 14, $R_1'$ is phenyl, $R_2'$ , $R_3'$ and $R_4'$ are hydrogen; and $R_5'$ is methyl.

N-(alk-2-ynyloxy) carbonylisoserine ester 14 is converted into oxazolidine 15 in presence of chemical such as alkoxyalkene or gem-dialkoxyalkane or 1,1,1-trialkoxyalkane. These chemicals are basically reagents for protection of vicinal functional groups such as diols or amino/amidoalchohols in cyclic form. The appropriate chemical is chosen on the basis of type of protecting group required. The most preferred alkoxylalkene, 2-methoxypropene, is available commercially. The reactions are catalysed by p-arylsulfonic acid or their pyridinium salt. The most preferred catalyst is pyridinium p-toluenesulfonate. The protection with 2-methoxypropene results in formation of 3-(alk-2-ynyloxy) carbonyl-5-oxazolidine carboxylic ester 15. In the most preferred oxazolidine ester, $R_1''$, $R_2''$ and $R_3''$ are hydrogen, $R_4''$ is phenyl; $R_5''$, $R_6''$ and $R_7''$ are methyl. 3-(Alk-2-ynyloxy) carbonyl-5-oxazolidine carboxylic ester is finally converted into corresponding acid by hydrolysis with alkali hydroxide or carbonate and mineral acid used successively. The hydrolysis is effected by mixing of a solution of ester in alcohol with an Aq. alkali hydroxide. The most preferred alcohol is methanol and the most preferred alkali hydroxide is sodium hydroxide. The mixing is done for 1 to 6 hr, the most preferably for 3 hr. After the hydrolysis is over, the reaction mixture is acidified to pH 3-6, most preferably 4.5. The acid 16 is isolated by extraction with dichloromethane. In the most preferred acid, $R_1''$, $R_2''$, and $R_3''$ are hydrogen, $R_4''$ is phenyl; $R_5''$ and $R_6''$ are methyl.

There is also provided a process of preparation of taxol and synthetic taxol analogs from 3-(alk-2-ynyloxy) carbonyl-5-oxazolidine carboxylic acids and taxane alcohols, which can be derived from natural or unnatural sources having the following reaction as an example:

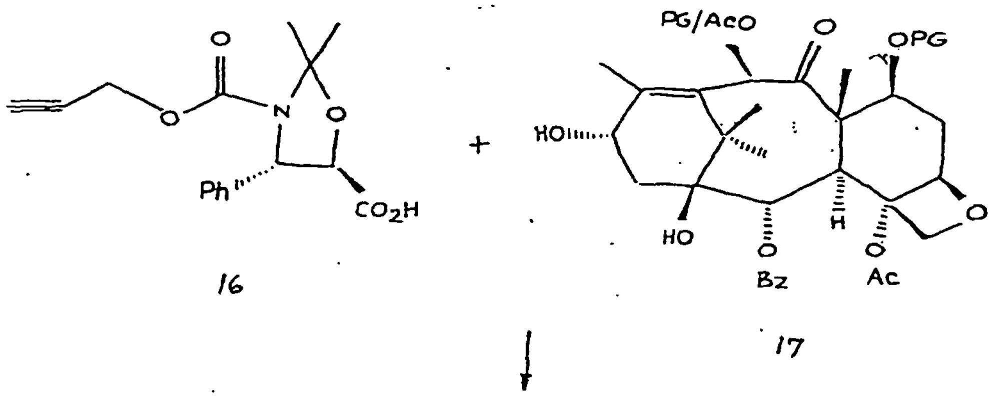

16

17

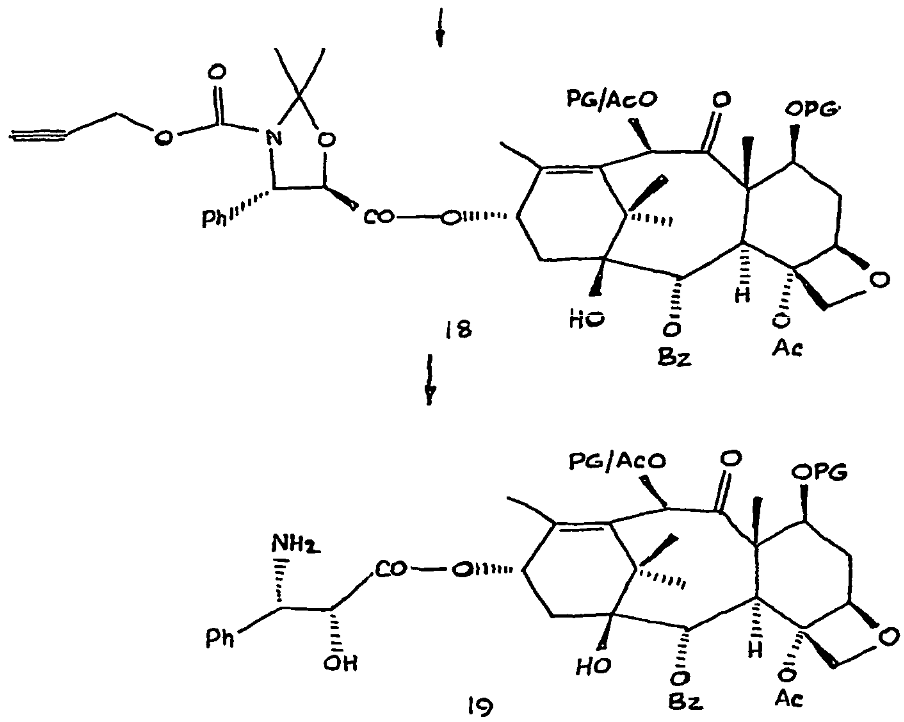

18

19

PG = Protecting groups

SCHEME - 2

The most preferred 3-(alk-2-ynyloxy) carbonyl-5-oxazolidine carboxylic is 3-(prop-2-ynyloxy) carbonyl-2,2-di methyl-4-phenyl-5-oxazolidine carboxylic acid. The preferred tetracyclic taxane alcohols are 7-O-triethylsilylbaccatin, 7-0-(2,2,2-trichloro -ethoxy) carbonylbaccatin, 7,10-di-O-(2,2,2-trichloroethoxy) carbonyl-10-deacetylbaccatin, 7, 10-di-O-benzyloxycarbonyl-10-deacetyl baccatin, 7-0-t-butyloxycarbonylbaccatin, 7, 10-di-O-t-butyloxycarbonyl-10-deacetylbaccatin, 7-O-(prop-2-ynyloxy) carbonyl baccatin, and 7,10-di-O-(prop-2-ynyloxy) carbonyl-10-deacetyl baccatin. The most preferred alcohols are 7-O-(2,2,2-trichloroethoxy) carbonylbaccatin, 7,10-di-O-(2,2,2-trichloroethoxy) carbonyl-10-deacetylbaccatin.

7-O-(2,2,2-trichloroethoxy) carbonylbaccatin, 7,10-di-O-(2,2,2-trichloroethoxy) carbonyl-10-deacetylbaccatin are synthesized from baccatin and 10-deacetylbacctin respectively in presence of 2,2,2-trichloroethoxy chloroformate and organic base. The preferred bases are pyridine, 4-dimethylaminopyridine or imidazole or like. The most preferred base is pyridine.

The 3-(prop-2-ynyloxy) carbonyl-2, 2-di methyl-4-phenyl-5 oxazolidine carboxylic acid 16 is converted into taxane ester 18 in presence of taxane alcohol such as 7-O-(2,2,2-trichloroethoxy) carbonylbaccatin 17 and condensing agent, preferably dicyclohexylcarbodiimde and organic base preferably 4-dimethylaminopyridine. This process can also be used to prepare various taxane esters 10 contemplated within the present invention from various 3-(alk-2-ynyloxy) carbonyl-5oxazolidine carboxylic acids and taxane alcohols.

The opening of the oxazolidine ring in ester 18 is effected by removal of prop-2-ynyloxy carbonyl with ammonium tetrathiomolybadate preferably benzyltriethyl ammonium tetrathiomolybadate which is prepared according to the known procedure described in Syn.Comm., 22(22), 3277-3284(1992). The removal of prop-2-ynyloxycarbonyl group is effected by exposing the mixture of ester and acetonitrile to ultrasound for 1-6 hr, most preferably for 3 hr. The reaction mixture is diluted with dichloromethane and then washed with water. Removal of the solvents gives the intermediate 19, free amine.

The resultant free amine can then be converted into taxol 1 or non-natural taxols such as 2 following the known methods as illustrated in scheme-3

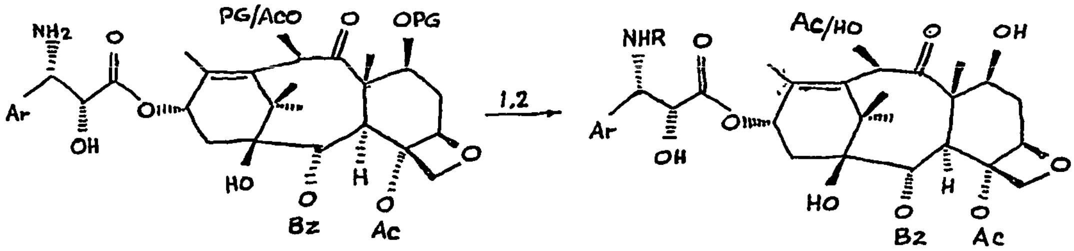


19 Ar = Ph, PG = $COOCH_2CCl_3$

Taxol Ar = Ph. R = Bz

1. Acylation of Amine
2. Deprotection of Protecting groups

SCHEME - 3

The invention will now be explained in greater details with the help of the accompanying examples:

Example 1:

(2R, 3S) phenylisoserine (25 gm, 0.138 mole) was dissolved in sodium bicarbonate and potassium bicarbonate (2.5 L) and then 0-5°C. Prop-2-ynyl chloroformate (25 gm, 0.21 mmol) was added drop wise into the solution in a period of 10 to 15 mins. After complete addition the reaction mixture was stirred for 6 Hrs at 05°C. pH of the solution was brought down to 4 by drop wise addition of 10% hydrochloric acid, at temperature 5-6°C. The acidified aqueous layer was extracted with a mixture of tetrahydrofuran and dichloromethane (4:1, 3 x 1L). The combined organic layer was washed with water until the neutral pH. The organic layer was dried over sodium sulphate and filtered evaporated in under vacuum below 40°C. The residue was dried in vacuum until constant weight to yield 20 g of compound 13.

Example 2:

To a ice cooled solution of Comp-13 (20 g, 76 mmol) in tetrahydrofuran (500 ml) was added drop wise a saturated ethereal solution of diazomethane. The addition was continued until the colour of diazomethane persists. Stirring was continued for further 2 Hrs. The excess diazomethane was removed by drop wise addition of acetic acid. The reaction mixture was then washed with 7% solution of sodium bicarbonate. The organic layer was washed with brine and dried over sodium sulfate. The organic layer was filtered and evaporated. The crude product was purified over silica gel (230-400) (eluent; hexane-ethyl acetate, 7:3 to 1:1) yield comp-14 (18 gm).

Example 3:

To a stirred solution of Comp 13 (5.0 g, 19 mmol) in tetrahydrofuran (50 ml) was added 1,1 carbonyl diimidazole (6.2 gm, 38 mmol) at 0-10°C. The stirring was continued for further 1 Hr. Methanol (3.2 ml) was then added drop wise into the solution in a period of 30 min. After complete addition, the reaction mixture was stirred for 2 Hr, when T.L.C. indicated completion of the reaction. The reaction mixture was concentrated and the residue dissolved in ethyl acetate (2 x 100 ml), washed with 5% hydrochloric acid, water and brine. The organic layer was dried and evaporated. The crude product was chromatograph over silica gel (eluent hexane : ethyl acetate 7:3 to 1:1) yield to comp 14 (4.5 gm).

Example 4:

To a stirred solution of Comp-14 (5g 18 mmol) in toluene (180 ml) was added 2-methoxypropene (35 ml, 0.36 mole), and pyridinium p-toluenesulfonate (425 mg). The mixture was stirred at 25°C for 10-15 min and then heated 80°C

for 6 Hr. Progress of the reaction was checked by T.L.C. After completion, the reaction, mixture was cooled and diluted with ethyl acetate (300 ml). The organic layer was washed with 5% sodium bicarbonate (2 x 125 ml) then with brine (150 ml). The organic layer was dried over sodium sulfate. After filtration, the organic layer was evaporated in rotavapour under vacuum below 40°C. The crude product was purified by column chromatography over silica gel, 240-400 mesh (eluent lower 85:15 hexane and ethyl acetate to yield comp-15, (3 g) as a colourless oil.

Example 5:

To a stirred solution of comp-15 (3.5 g, 11 mmol) in methanol (35 ml) was added a solution of lithium hydroxide (1.8 g) in water (15 ml). After stirring at 20-25°C for 3 Hr (absence of starting material, was checked by T.L.C.). The reaction mixture was concentrated to reduce the volume to 15 ml and diluted with dichloromethane (200 ml). The mixture was then washed with water (250 ml). The aqueous layer was separated and cooled to 5-15°C and solution pH was brought to 4 by adding 10% hydrochloric acid. The aqueous layer was extracted with dichloromethane (3 x 300 ml). The combined organic layer was washed with brine and dried over, preferably sodium sulfate and magnesium sulfate. After filtration the organic layer was evaporated under vaccum and residue was dried up to constant weight to yield compound - 16 (2.2 gm).

Example 6:

To a stirred solution of comp-16 (2.0 g, 6.6 mmol) in toluene or (20 ml) was added dicyclohexylcarbodimide (3g) followed by addition of 7-0-trichloroethoxycarbonylbaccatin 17, (2 g, 2.62 mmol) and DMAP (101 mg). The stirring was continued for 1 Hr. The reaction mixture was diluted with ethyl acetate (100 ml) and filtered. The filtrate was washed successively with aqueous saturated sodium bicarbonate and brine. The organic layer was dried and evaporated under vaccum. The crude product was purified over silica gel (eluent 4:1 hexane and ethyl acetate) to yield comp-18 (1.9 g).

Example-7

A mixture of comp-18 (2 g, 1.91 mmol) and benzyl triethylammonium tetrathiomolybedate (1.16 g) in acetonitrile (5 ml) was kept in ultrasound bath for 4 hr and then diluted with dichloromethane (30 ml). The mixture was washed with water (2x10 mL). The organic layer was evaporated to give comp-19 (1.75 g) as solid.

Example-8

A mixture of comp-19 (1 g, 1.08 mmol) in ethyl acetate (5 mL), and saturated Aq. sodium bicarbonate (3 mL) was added benzoyl chloride (0.17 mL) in ethyl acetate (2 mL) drop wise at 0°C. After, the complete addition, the temperature was raised to 25°C and the stirring was continued for further 2 hr. The Aq. layer was separated and the organic layer was washed with water until neutral. The organic layer was dried over sodium sulfate and then evaporated to give a solid. A mixture of this solid, acetic acid (10 mL), methanol (5 mL) and zinc powder (1 g) was heated to 60°C with stirring. After 1 hr, the reaction mixture was cooled and then filtered. The filtrate was evaporated. The residue was dissolved in ethyl acetate and then washed with Aq. sodium bicarbonate, water and brine. The organic layer was dried over sodium sulfate, then filtered and evaporated. The solid residue was crystallised with acetone-hexane then with methanol- water to afford Taxol (0.74 g, yield 80% from comp-18 99% HPLC w/w assay, 99% HPLC chromatographic purity).

## Claims

1. Oxazolidine carboxylic acid having formula

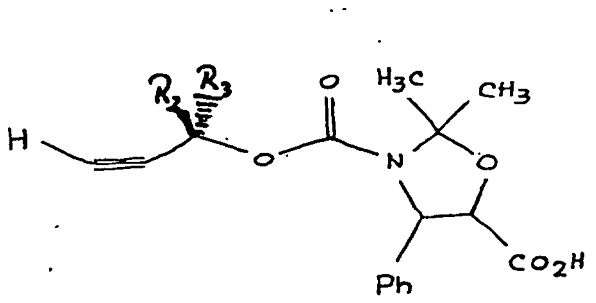

9

wherein $R_2$ and $R_3$ are hydrogen.

**2.** A process for the preparation of oxazolidine carboxylic acid as claimed in claim 1 having the following reaction:

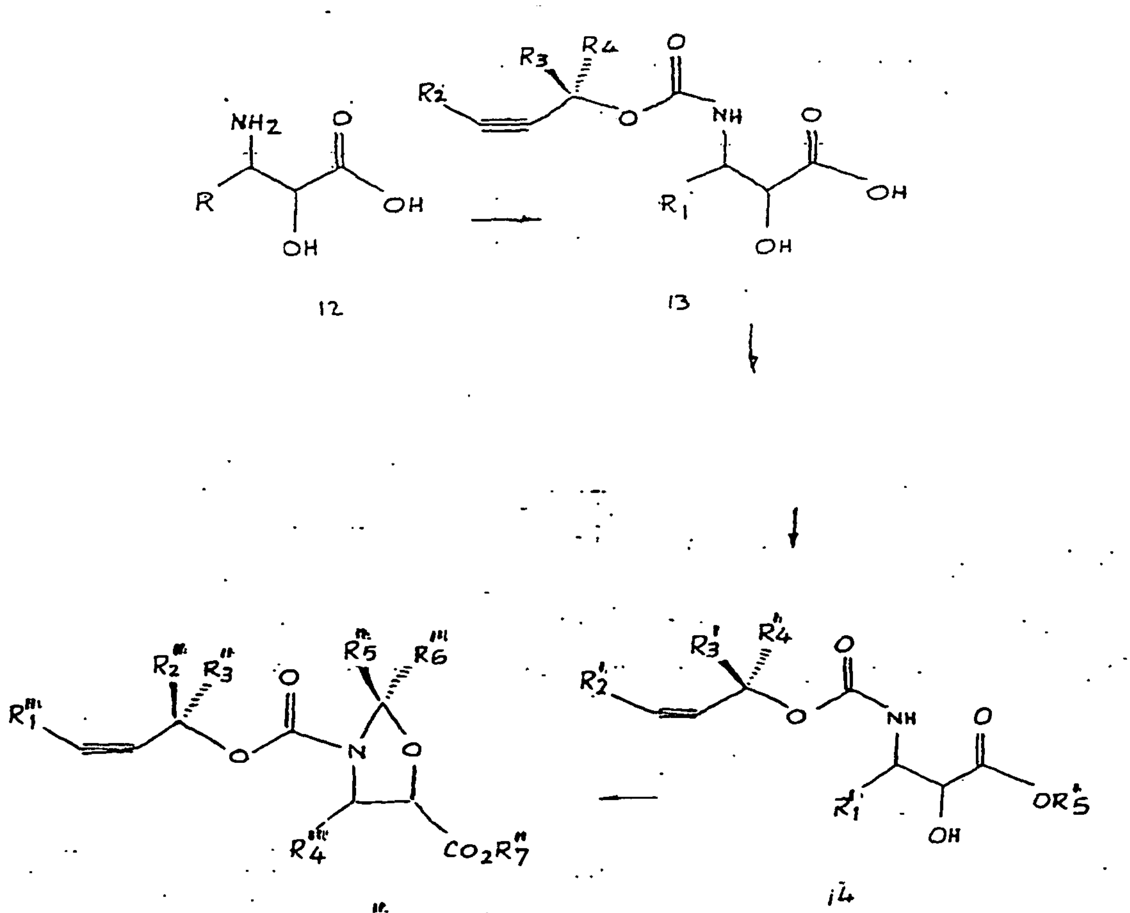

12

13

14

15 $R_7''' = Alkyl$

16 $R_7''' = H$

and wherein isoserine of formula 12 is condensed with alk-2-ynyl haloformate in the presence of a base to produce N-(alk-2-ynyloxy) carbonylisoserine of formula 13, converting said carbonylisoserine into corresponding ester of formula 14 in the presence of alcohol, converting the ester into oxazolidine ester of formula 15, converting said ester by ester hydrolysis to produce the corresponding acid of formula 16, wherein

R is aryl or phenyl , $R_1$ is phenyl ,$R_2$,$R_3$, and $R_4$ are hydrogen, $R_1'$ is phenyl , $R_2'$, $R_3'$ and $R_4'$ are hydrogen

and $R_5'$ is methyl, $R_1''$, $R_2''$ and $R_3''$ are hydrogen, $R_4''$ is phenyl, $R_5''$ and $R_6''$ are methyl

3. A process for preparation of taxol and synthetic taxols having the following reaction:

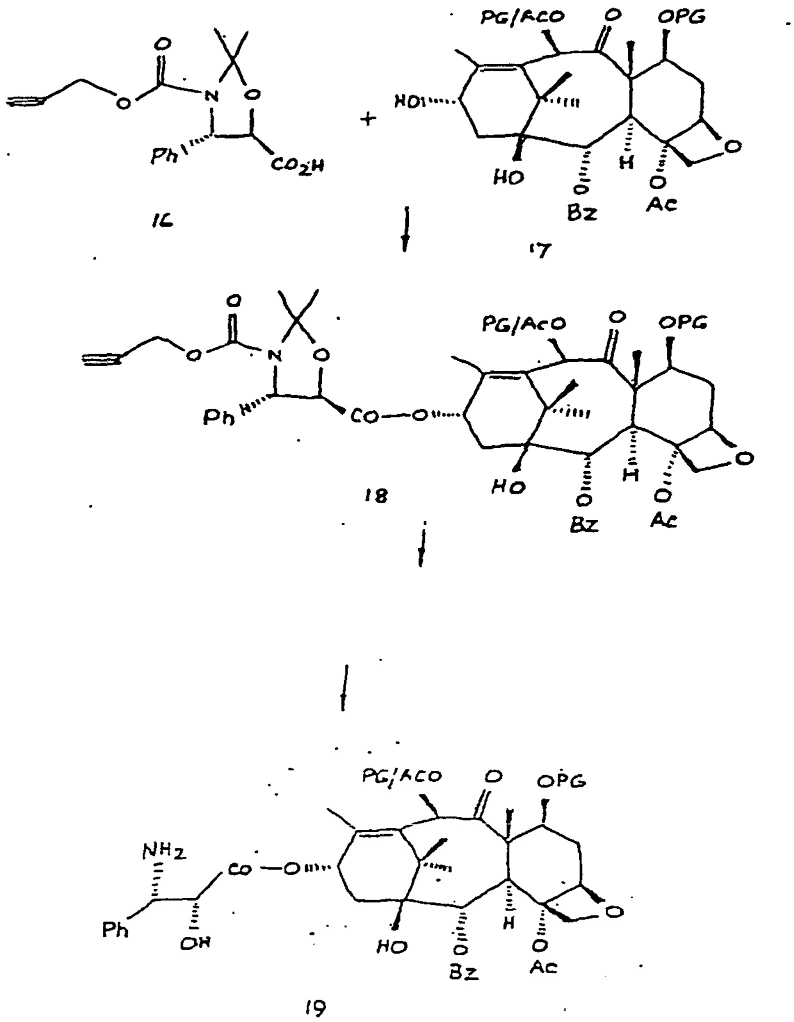

16 17 18 19

PG = Protecting groups

And wherein oxazolidine carboxylic acid of formula 16 is condensed with taxane alcohol of formula 17 using a condensing agent and an organic base to produce the ester of formula 18 which is then treated with tetrathiomolybdate, to produce an amine of formula 19. The amine 19 is then converted into taxol and synthetic taxol analogs using known procedures.

4. A process as claimed in claim 3, wherein the said condensing agent is preferably dicyclohexylcarbodiimide.

5. A process as 'claimed in claim 3, wherein the said organic base is preferably 4-dimethylaminopyridine.

6. A process as claimed in claim 3, wherein said taxane alcohol is 7-O-(2, 2, 2-trichloroethoxy) carbonylbaccatin.

7. A process as claimed in claim 3, wherein said prop-2-ynyloxy carbonyl group cleaving reagent is benzyltriethylammonium tetrathiomolybdate.

## Patentansprüche

1. Oxazolidincarbonsäure mit der Formel

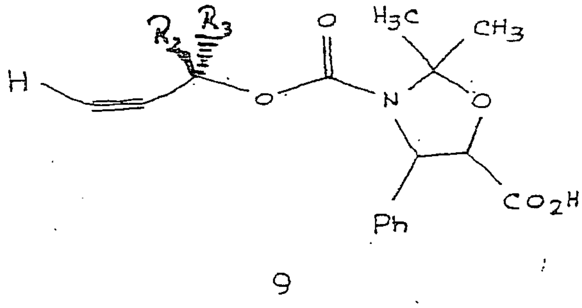

9

wobei $R_2$ und $R_3$ Wasserstoff sind.

2. Ein Verfahren zur Herstellung von Oxazolidincarbonsäure gemäß Anspruch 1 mit der folgenden Reaktion

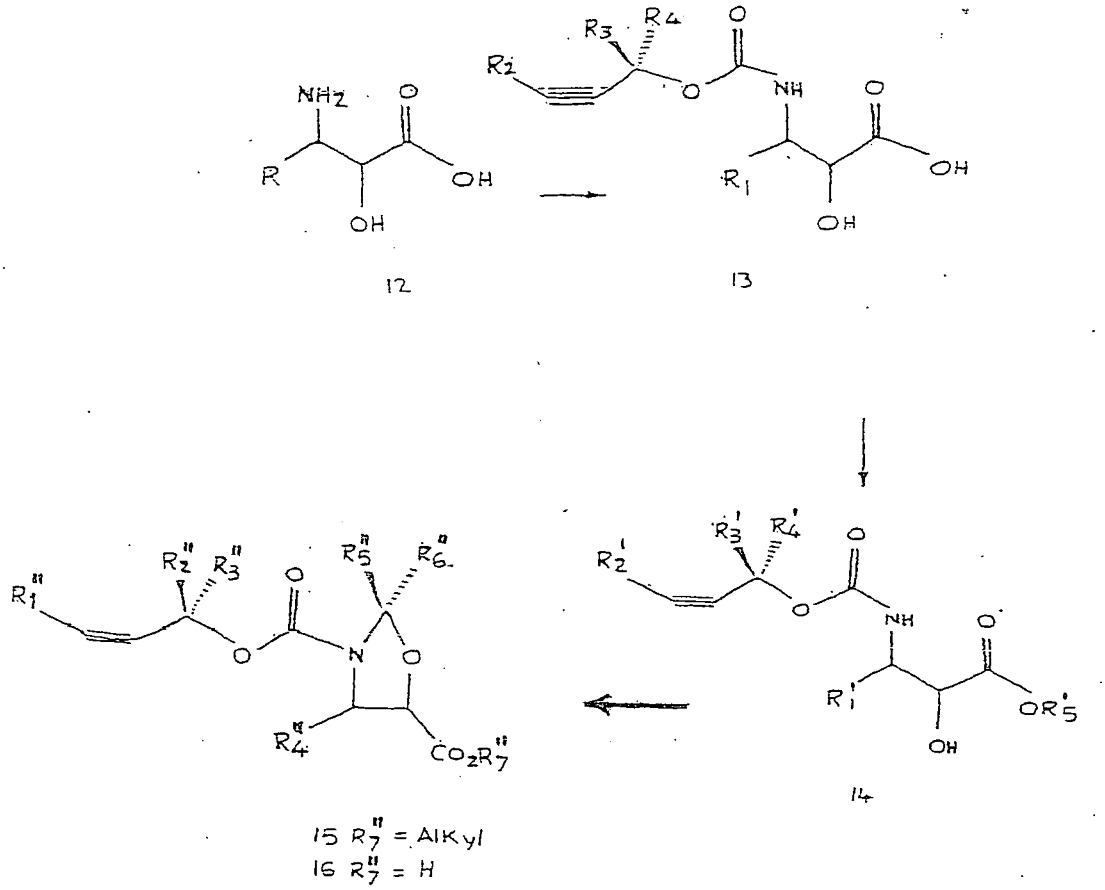

12

13

14

15 $R_7'' =$ Alkyl

16 $R_7'' =$ H

wobei das Isoserin der Formel 12 kondensiert wird mit alk-2-ynyl Haloformat in der Anwesenheit einer Base, um N-(alk-2-ynyloxy) Carbonisoserin der Formel 13 herzustellen, Umwandlung des genannten Carbonisoserins in das

entsprechende Ester gemäß Formel .14 in der Anwesenheit von Alkohol, Umwandlung des Esters in Oxazolidinester der Formel 15, Umwandlung des genannten Esters durch Esterhydrolyse, um die entsprechende Säure der Formel 16 herzustellen, wobei R Aryl oder Phenyl ist, $R_1$ Phenyl ist, $R_2$, $R_3$ und $R_4$ sind Wasserstoff, $R_1$' ist Phenyl, $R_2$', $R_3$' und $R_4$' sind Wasserstoff und $R_5$' ist Methyl, $R_1$", $R_2$" und $R_3$" sind Wasserstoff, $R_4$" ist Phenyl, $R_5$" und $R_6$" sind Methyl.

**3.** Ein Verfahren zur Herstellung von Taxol und synthetischen Taxolen mit der folgenden Reaktion:

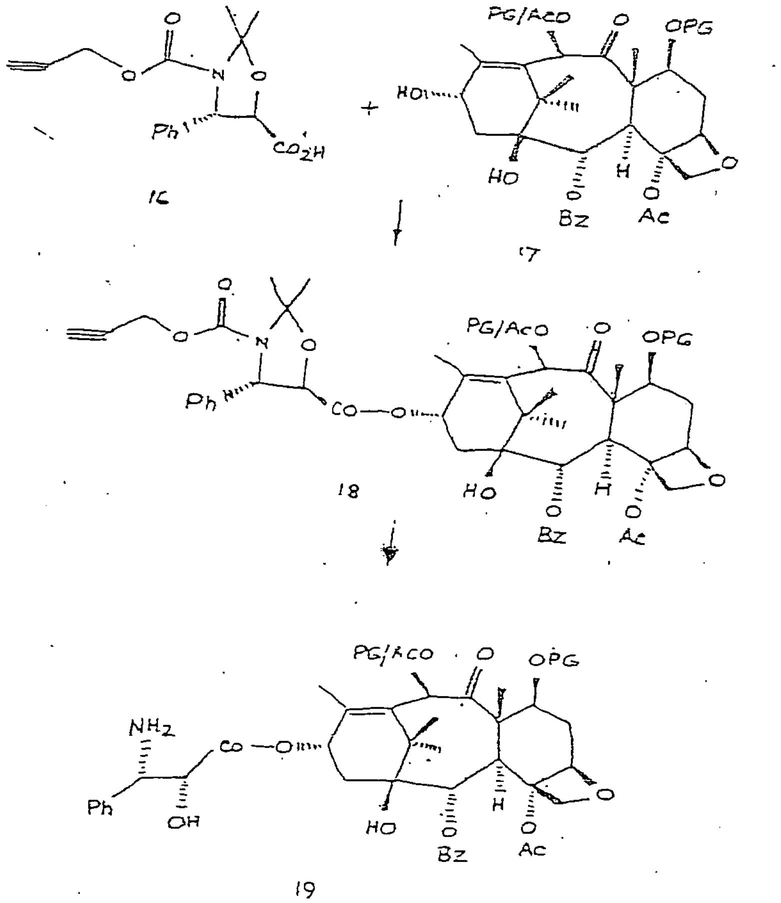

16

17

18

19

PG = Schutzgruppe

und wobei Oxazolidincarbonsäure der Formel 16 kondensiert wird mit Taxanalkohol der Formel 17 unter Benutzung eines Kondensierungshilfsmittels und einer organischen Base, um das Ester der Formel 18 herzustellen, das dann behandelt wird mit Tetrathiomylbdat, um ein Amin der Formel 19 herzustellen, wobei das Amin 19 dann umgewandelt wird in Taxol und synthetische Taxolanaloge unter Benutzung bekannter Verfahren.

**4.** Ein Verfahren gemäß Anspruch 3,
wobei das besagte Kondensierungsmittel vorzugsweise Dicyclohexylcarbodiimid ist.

**5.** Ein Verfahren gemäß Anspruch 3,
wobei die genannte organische Base vorzugsweise 4-Dimethylaminopyridin ist.

6. Ein Verfahren gemäß Anspruch 3, wobei der genannte Taxanalkohol 7-0 (2,2,2-Trichloroethoxy) Carbonylbaccatin ist.

7. Ein Verfahren gemäß Anspruch 3, wobei das genannte prop-2-ynyloxy Carbon-Gruppen Spaltmittel Benzyltriethylammoniumtetrathiomolybdat ist.

**Revendications**

1. Acide carboxylique d'oxazolidine de formule

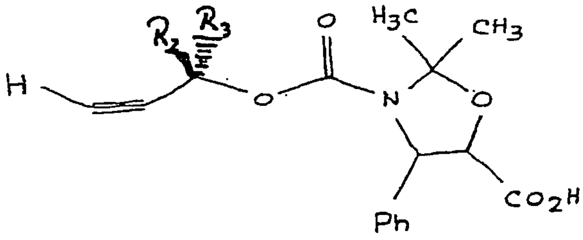

9

dans lequel $R_2$ et $R_3$ sont un hydrogène.

2. Procédé de préparation d'un acide carboxylique d'oxazolidine selon la revendication 1 ayant la réaction suivante :

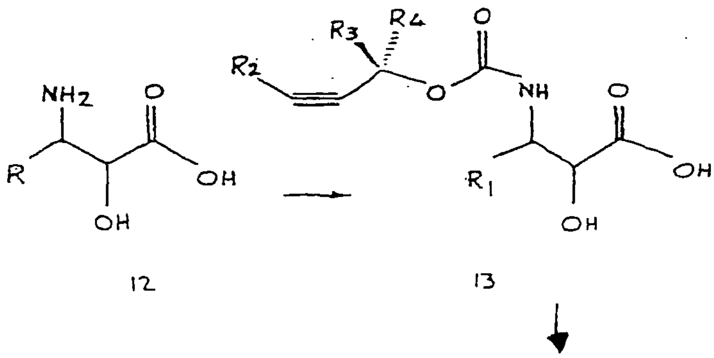

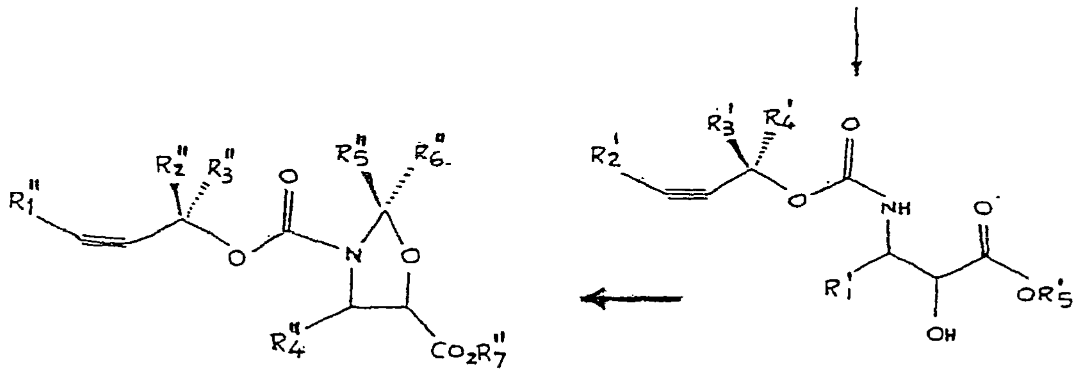

14

15 $R_7'' = $ Alkyl
16 $R_7'' = H$

et dans lequel l'isosérine de formule 12 est condensée avec un haloformate d'alk-2-ynyle en présence d'une base pour produire une N-(alk-2-ynyloxy) carbonylisosérine de formule 13, en convertissant ladite carbonylisosérine en ester correspondant de formule 14 en présence d'un alcool, en convertissant l'ester en ester d'oxazolidine de formule 15, en convertissant ledit ester par une réaction d'hydrolyse d'ester pour produire l'acide correspondant de formule 16, dans lequel
R est un aryle ou un phényle, $R_1$ est un phényle, $R_2$, $R_3$ et $R_4$ sont un hydrogène, $R_1$' est un phényle, $R_2$', $R_3$' et $R_4$' sont un hydrogène et $R_5$' est un méthyle, $R_1$'', $R_2$'' et $R_3$'' sont un hydrogène, $R_4$'' est un phényle, $R_5$'' et $R_6$'' sont un méthyle.

**3.** Procédé de préparation de taxol et de taxols synthétiques ayant la réaction suivante :

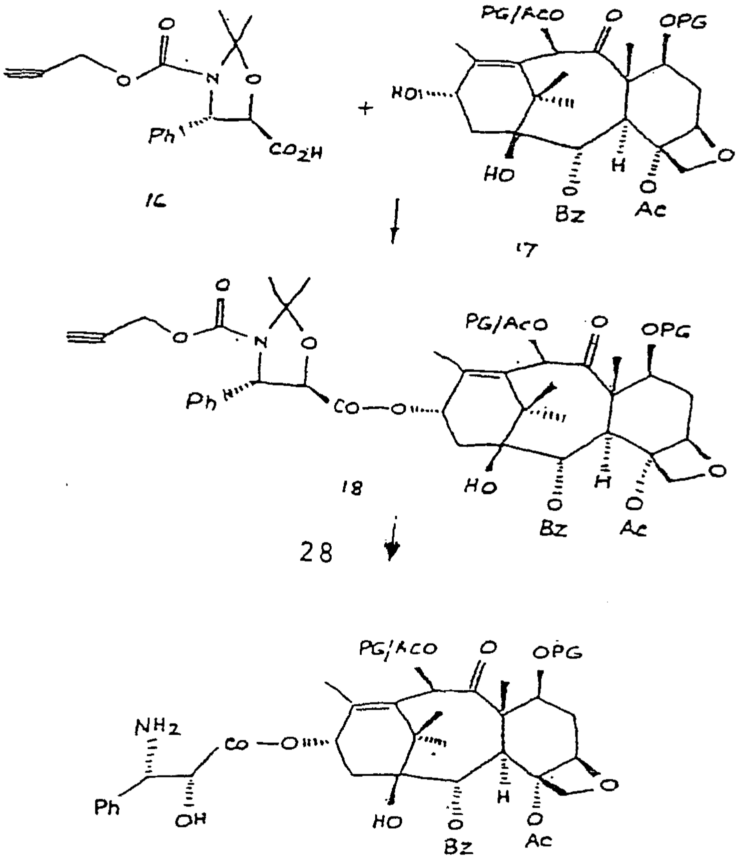


16 17 18 19

PG = groupes protecteurs

et dans lequel l'acide carboxylique d'oxazolidine de formule 16 est condensé avec l'alcool de taxane de formule 17 en utilisant un agent de condensation et une base organique pour produire l'ester de formule 18 qui est ensuite traité avec un tétrathiomolybdate pour produire une amine de formule 19, l'amine de formule 19 étant ensuite convertie en taxol et en analogues de taxol synthétiques en utilisant des procédures connues.

4. Procédé selon la revendication 3, dans lequel ledit agent de condensation est de préférence le dicyclohexylcarbodiimide.

5. Procédé selon la revendication 3, dans lequel ladite base organique est de préférence la 4-diméthylaminopyridine.

6. Procédé selon la revendication 3, dans lequel ledit alcool de taxane est la 7-O-(2,2,2-trichloroéthoxy) carbonylbaccatine.

7. Procédé selon la revendication 3, dans lequel ledit réactif de clivage du groupe prop-2-ynyloxy carbonyle est le tétrathiomolybdate de benzyltriéthylammonium.